# DEMANDE DE BREVET EUROPEEN

(11) **EP 0 604 307 A1**
(43) Date de publication de la demande: **29.06.1994**
(21) Numéro de dépôt: 93403127.9
(22) Date de dépôt: 21.12.1993
(51) Int. Cl.: G01N 33/543, G01N 33/18

(54) **Phase solide, dispositif et procédé de détection périodique d'un composé dilué**

(30) Priorité: 22.12.1992 FR 9215461
(71) Demandeur: TRANSIA-DIFFCHAMB S.A., F-69007 Lyon (FR); LYONNAISE DES EAUX - DUMEZ, F-92000 Nanterre (FR); SOCIETE D'ETUDE ET DE REALISATION D'EQUIPEMENTS SPECIAUX - S.E.R.E.S. Société anonyme dite:, F-13290 - Les Milles (FR)
(72) Inventeur: Drouet, Xavier, F-69002 Lyon (FR); de Solan, Marie-Noelle, F-69002 Lyon (FR); Rybacki, Danièle, F-78400 Chatou (FR); Laugier, Francis, F-13770 Venelles (FR)
(74) Mandataire: Warcoin, Jacques

(57) **Abrégé**

L'invention concerne un procédé de détection périodique d'un composé notamment l'atrazine, dilué dans un échantillon liquide, par un procédé de type immunologique mettant en oeuvre des anticorps ou autres éléments de reconnaissance spécifique du composé, caractérisé en ce que :
(a) on met en présence, dans une chambre réactionnelle (1) pendant un temps déterminé, une phase solide fluide (9) non solidaire de ladite chambre formée d'un support solide sur lequel sont immobilisés les anticorps ou autres éléments de reconnaissance spécifique et l'échantillon liquide (11),
b) la phase solide fluide est alors séparée de l'échantillon, éventuellement lavée et transférée dans une chambre de révélation,
c) la présence et/ou la quantité de composé fixé sur la phase solide fluide sont révélées par des moyens appropriés et éventuellement dosées quantitativement,
d) en ce qu'après que la phase solide fluide qui vient d'être utilisée a été évacuée de la chambre réactionnelle, elle est remplacée par une nouvelle phase solide pour recommencer l'opération précédente.

L'invention concerne également un dispositif pour mettre en oeuvre le procédé et la phase solide fluide destinée à être utilisée dans ledit procédé.

## Description

L'invention concerne un procédé de détection périodique d'un composé, dilué dans un échantillon liquide, par un procédé de type immunologique mettant en oeuvre des anticorps ou autres éléments de reconnaissance spécifique du composé et une phase solide fluide destinée à être utilisé pour mettre en oeuvre ledit procédé.

Elle concerne également un dispositif de détection pour mettre en oeuvre ledit procédé.

Tout au long de la chaîne de production, les aliments peuvent être contaminés par des microorganismes ou des substances chimiques indésirables. Dans le secteur des industries agro-alimentaires, les méthodes de contrôle de la qualité et de l'inocuité des produits doivent être suffisamment fiables, sensibles et rapides pour s'intégrer parfaitement à des procédés de fabrication de plus en plus complexes et sophistiqués. Ainsi, des capteurs en ligne appropriés peuvent être très utiles pour surveiller régulièrement les matériaux mis en oeuvre, et plus particulièrement les matériaux liquides.

Par ailleurs, les progrès accomplis dans le domaine de l'immunologie appliquée ont débouché sur la mise au point de nombreux tests de diagnostic (hépatite, SIDA...) et de nombreuses techniques de titration (hormones, toxines...) parmi lesquels certains ont été mis au point pour le secteur des industries agro-alimentaires (salmonelles, aflatoxines, enterotoxines de staphylocoques, pesticides...).

Dans le cas particulier des eaux de consommation courantes, compte tenu des normes de plus en plus sévères mises en vigueur, il est nécessaire de trouver le moyen de surveiller périodiquement la qualité de ces eaux et notamment de repérer la présence de contaminants qui rendent, à une certaine concentration, l'eau impropre à la consommation. Il est par exemple bien connu que les nappes phréatiques peuvent être contaminées du fait de leur rémanence, par les triazines notamment l'atrazine qui sont des herbicides couramment employés par les agriculteurs. Par exemple, il suffit d'un orage violent pour que les eaux pluviales entraînent brusquement une quantité importante d'atrazine dans les nappes phréatiques et par voie de conséquence, dans les eaux destinées à la consommation. Il est donc clair qu'il existe un besoin pour un procédé de détection permettant d'éviter ces accidents.

Ainsi, un des objets de la présente invention est de proposer un procédé, ainsi qu'un dispositif assurant la mise en oeuvre du procédé, permettant une détection périodique et automatique d'un contaminant, tel qu'un pesticide comme l'atrazine, par exemple au rythme de 10 à 40 mesures par jour, sur plusieurs échantillons liquides d'un volume de plusieurs millilitres et avec une autonomie de fonctionnement de l'ordre de plusieurs jours.

Ce procédé permet d'atteindre une sensibilité de l'ordre d'une ou quelques centaines de picogrammes par millilitre.

Selon un mode général de réalisation, le procédé de détection périodique d'un composé, dilué dans un échantillon liquide, est caractérisé en ce que :
(a) on met en présence dans une chambre réactionnelle pendant un temps déterminé, une phase solide fluide non solidaire de ladite chambre, sur laquelle sont immobilisés les anticorps ou autres éléments de reconnaissance spécifique et l'échantillon liquide,
b) la phase solide fluide est alors séparée de l'échantillon, éventuellement lavée et transférée dans une chambre de rélévation,
c) la présence et/ou la quantité de composé fixé sur la phase solide fluide sont révélées par des moyens appropriés et éventuellement dosées quantitativement,
d) en ce qu'après que la phase solide fluide qui vient d'être utilisée a été évacuée de la chambre réactionnelle, elle est remplacée par une nouvelle phase solide fluide pour recommencer l'opération précédente.

Ainsi, contrairement au procédé couramment utilisé dans l'art, celui selon la présente invention utilise comme moyen central une phase solide fluide, ce qui présente des avantages considérables dans le cas d'une automatisation.

Par l'expression phase solide fluide, on entend désigner un support solide formé de particules discrètes associées aux anticorps ou autres éléments de reconnaissance spécifique.

Par l'expression temps déterminé, on désignera le temps nécessaire pour permettre à la réaction immunologique de s'effectuer.

L'invention s'est avérée particulièrement utile dans la détection des contaminants comme les pesticides dans les eaux de consommation courante.

Elle peut être néanmoins appliquée à d'autres substances et trouve par exemple une utilisation dans la numération ou la détection des germes à conditions d'enrichir l'échantillon préalablement.

Suivant le procédé selon l'invention, la phase solide fluide est transférée dans une autre chambre réactionnelle dans laquelle s'effectue l'étape c) de révélation. Cette variante est industriellement aisée à mettre en oeuvre.

De façon générale, le test de détection consiste à mesurer une différence par rapport à un témoin déterminé. Cela nécessite donc d'établir préalablement des courbes étalon avec un liquide témoin par exemple l'eau distillée afin de pouvoir établir la correspondance avec la solution à tester.

La méthode de type immunologique fait intervenir notamment la réaction immuno-enzymatique fondamentale bien connue de l'homme du métier.

Il s'agit par exemple de la méthode ELISA dont la mise en oeuvre peut être différente selon le mode opératoire choisi. On pourra ainsi utiliser la méthode sandwich directe, ou la méthode de type compétition.

Selon la méthode sandwich, le procédé de détection consiste à mettre en présence la phase solide fluide, sur laquelle se sont éventuellement fixés les composés, avec un conjugué lié à une enzyme spécifique d'un substrat et d'un chromogène, puis à faire réagir ledit substrat et ledit chromogène, introduit par exemple sous forme de solution, avec l'enzyme associé au conjugué, puis ensuite à doser par colorimétrie la solution. Par comparaison avec une solution témoin, on peut déterminer ainsi la présence éventuelle et la quantité de contaminants présents dans le liquide à tester.

Selon un mode de réalisation, le conjugué lié à une enzyme est un anticorps spécifique du composé. Dans ce cas, l'ajout d'un substrat et d'un chromogène au cas où le liquide à tester contenait des composés va provoquer la coloration du liquide. Le liquide est ensuite transféré dans une cuve appropriée à la mesure de la densité optique.

Selon la méthode de type compétition, le conjugué lié à une enzyme est le composé à détecter, ce qui va provoquer un équilibre entre le composé éventuel provenant du liquide à tester et le contaminant lié à l'enzyme. L'ajout d'un substrat et d'un chromogène va permettre de doser par colorimétrie la solution selon un procédé inverse de la méthode précédente dans la mesure où une solution colorée au maximum indiquera une absence de composé.

Néanmoins, l'invention n'est pas limitée à ce procédé de détection particulier. Ainsi, la réaction immunologique peut faire intervenir d'autres éléments de reconnaissance que les anticorps comme une enzyme ou du DNA, ceux-ci pouvant également être greffés sur le support.

De préférence, les anticorps spécifiques sont greffés sur le support selon un procédé connu. Selon la nature du support, le mode de fixation sera une adsorption covalente ou non. La phase solide fluide est avantageusement constituée de billes ou microbilles bien connues.

Parmi les supports convenant pour la présente invention, on peut citer à titre indicatif les matériaux suivants : le polystyrène, le nylon, le verre, le téflon, le polyéthylène, le polypropylène, l'acétate de cellulose, le polyacrylamide.

La densité d'anticorps par unité de surface du support solide sera de préférence comprise entre 0,1 et 1 µg/cm².

Le procédé de détection est particulièrement adapté à la détection de contaminants, notamment les pesticides comme les triazines dans les liquides susceptibles d'en contenir comme les eaux de surface, les eaux usées et les eaux potables.

Selon un mode de réalisation préféré, le procédé de détection est caractérisé par les étapes suivantes :
a) on met en présence dans une cuve réactionnelle, un mélange d'un liquide susceptible de contenir lesdits contaminants et un liquide contenant une quantité déterminée d'un conjugué constitué par ledit contaminant lié à une enzyme spécifique du substrat chromogène et une phase solide fluide, associée à des anticorps spécifiques du contaminant à détecter,
b) on évacue lesdits liquides,
c) on transfère la phase solide fluide dans une cuve de révélation,
d) on introduit le substrat et le chromogène dans la cuve de révélation,
e) la réaction de révélation est arrêtée,
f) on mesure la densité optique,
g) on évacue la phase solide fluide ,
i) éventuellement une autre phase solide fluide est introduite dans la cuve réactionnelle.

Ce procédé présente l'avantage de rendre l'automatisation particulièrement aisée.

Il est préférable entre les étapes b) et c) de rincer la cuve avec un liquide approprié.

De préférence donc, le procédé est automatisé de façon à permettre une détection en continu ou semi-continu. Dans le cas d'un enrichissement préalable de l'échantillon, cette étape préalable pourra également être automatisée.

L'invention a également pour objet une phase solide fluide associée à des anticorps ou autres éléments de reconnaissance spécifique décrit ci-avant destinée à être utilisée dans le procédé qui vient d'être décrit.

De préférence, la phase solide fluide sera associée à des anticorps spécifiques des triazines notamment de l'atrazine.

L'invention concerne également un dispositif de détection pour mettre en oeuvre le procédé précédemment décrit, caractérisé en ce qu'il comprend une cuve réactionnelle pourvue de moyens d'acheminement :
de la phase solide fluide,
de l'échantillon liquide à tester,
du conjugué lié à une enzyme spécifique d'un substrat chromogène,
éventuellement d'eau de lavage
pourvue de moyens d'évacuation,
de la phase solide fluide,
du milieu liquide.
la phase solide fluide est transférée dans une cuve de révélation pourvue de moyens d'acheminement :
de la phase solide fluide,
d'une solution de substrat,
d'une solution de chromogène,
d'une solution d'arrêt de réaction
de moyens d'évacuation,
de la phase solide fluide,
du milieu liquide
de moyens de mesure de la densité optique du milieu liquide.

De préférence, la phase solide fluide est transférée de la cuve réactionnelle à la cuve de révélation au moyen d'un fond amovible de la cuve réactionnelle.

De préférence, I'évacuation du milieu liquide est assistée d'une pompe péristaltique.

L'invention est maintenant illustrée par un exemple de réalisation particulier donné à titre indicatif.

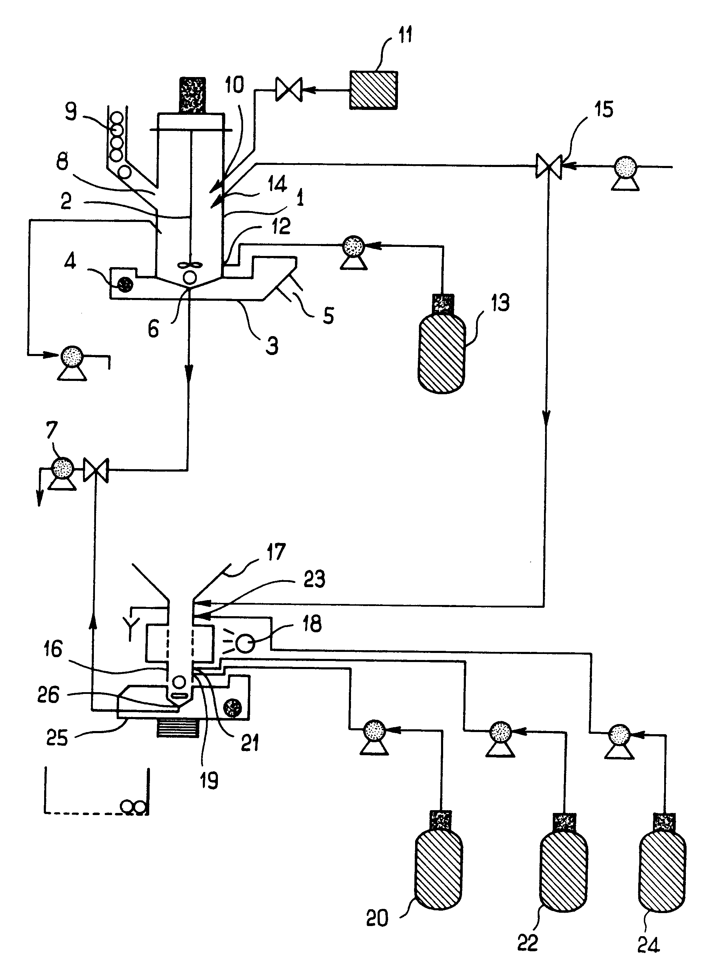

La figure unique illustre un schéma de dispositif permettant de mettre en oeuvre le procédé qui va être décrit.

Une cuve réactionnelle 1, munie d'un agitateur 2 est pourvue d'un fond 3 amovible qui pivote autour d'un axe 4 perpendiculaire à l'axe de la cuve 1 et situé en dehors du prolongement du volume défini par la cuve. Ce fond est actionné par un vérin (seulement symbolisé) 5 et obture de façon étanche la base de la cuve en position fermée. Il est pourvu dans l'axe de la cuve d'un orifice de sortie 6 reliée à une pompe péristaltique 7.

Cette cuve est munie au niveau des bords longitudinaux d'une entrée 8 de la phase solide fluide 9 qui est formée d'un support solide sur lequel ont été greffés des anticorps spécifiques de l'atrazine par exemple, par un procédé bien connu de l'homme du métier.

La cuve comporte également une entrée 10 pour l'échantillon à tester 11 provenant d'une source ayant été contaminée par l'atrazine, une entrée 12 pour une solution 13 déterminée d'un conjugué constitué d'atrazine associé à la peroxydase, une entrée 14 d'eau déminéralisée 15 de rinçage. Les entrées 12, 14 sont reliées à des pompes d'alimentation.

L'acheminement de la phase solide fluide peut être assuré par un dispositif d'électro-aimant.

Dans le prolongement inférieur de la cuve réactionnelle est située une cuve de révélation 16 thermostatée munie d'un entonnoir 17 à son sommet destinée à recevoir du fait de la gravité, la phase solide fluide 9 de la cuve réactionnelle 1, une fois l'incubation effectuée et le fond amovible en position ouverte.

Une cellule de détection 18 est placée à côté de ladite cuve 16 pour pemettre une révélation automatique de la densité optique.

La cuve de révélation est pourvue d'une entrée 19 pour le substrat 20, d'une entrée 21 pour le chromogène 22, d'une entrée 23 pour l'acide sulfurique 24 et d'une entrée pour l'eau de rinçage.

De la même façon que pour la cuve réactionnelle, le fond 25 de la cuve est amovible pour permettre l'évacuation de la phase solide fluide et un orifice de sortie 26 est présent au milieu de ce fond, relié à une pompe péristaltique 7.

Selon cet exemple de réalisation, on utilise une phase solide fluide constituée de billes de polystyrène de densité 1,02 de diamètre 6,4 mm et de poids 0,14 g.

1 ml de conjugué atrazine-peroxydase sont mélangés sous agitation dans la cuve réactionnelle à 20 ml d'échantillon à tester provenant d'une source ayant été contaminée par l'atrazine, puis on introduit 0,14 g de phase solide fluide et on laisse l'ensemble incuber 15 min.

Le liquide est évacué par la sortie 6 puis les billes sont rincées par de l'eau déminéralisée provenant de l'entrée 14.

Le vérin hydraulique 5 fait pivoter le fond 3 et les billes tombent dans la cuve de révélation 16 où l'on achemine la solution de substrat par l'entrée 19, la solution de chromogène par l'entrée 21. Au contact du support, l'enzyme fixée sur les billes réagit avec le substrat qui, transformé, réagit avec le chromogène, ce qui conduit à la coloration de la solution. La réaction est stoppée par l'introduction d'acide sulfurique 24 de manière connue. Une fois la densité optique mesurée à une longueur d'onde donnée (470 mm) et comparée à une courbe témoin, la détection est achevée. Les billes sont évacuées par pivotage du fond 25 et la solution est éliminée par pompage. La cuve est lavée.

Une fois l'opération terminée, une nouvelle opération peut à nouveau débuter.

L'invention n'est pas limitée à ce procédé de détection particulier qui vient d'être décrit, mais au contraire, elle s'étend à d'autres procédés faisant intervenir d'autres réactifs (enzyme, DNA...). Enfin, cette méthode peut être utilisée pour la numération ou la détection des germes à condition d'enrichir l'échantillon préalablement, cette étape pouvant elle aussi être automatisée.

## Revendications

1. Procédé de détection périodique d'un composé, dilué dans un échantillon liquide, par un procédé de type immunologique mettant en oeuvre des anticorps ou autre élément de reconnaissance spécifique du composé, caractérisé en ce que :
(a) on met en présence, dans une chambre réactionnelle (1) pendant un temps déterminé, une phase solide fluide (9) fluide non solidaire de ladite chambre sur laquelle sont immobilisés les anticorps ou autres éléments de reconnaissance spécifique et l'échantillon liquide (11),
b) la phase solide fluide est alors séparée de l'échantillon, éventuellement lavée et transférée dans une seconde chambre de révélation (16),
c) la présence et/ou la quantité de composé fixé sur la phase solide fluide sont révélées par des moyens appropriés et éventuellement dosées quantitativement,
d) en ce qu'après que la phase solide fluide qui vient d'être utilisée a été évacuée de la chambre réactionnelle, elle est remplacée par une nouvelle phase solide fluide pour recommencer l'opération précédente.

2. Procédé de détection périodique selon la revendication 1, caractérisé en ce que la révélation est effectuée par un procédé de type ELISA.

3. Procédé de détection périodique selon la revendication 2, caractérisé en ce que la révélation est effectuée en utilisant la technique sandwich.

4. Procédé de détection périodique selon la revendication 2, caractérisé en ce que la révélation est effectuée par un dosage de compétition.

5. Procédé de détection selon la revendication 4, caractérisé en ce que :
a) on met en présence dans une cuve réactionnelle, un mélange d'un liquide susceptible de contenir lesdits contaminants et un liquide contenant une quantité déterminée d'un conjugué constitué par ledit contaminant lié à une enzyme spécifique du substrat chromogène et une phase solide fluide, associé à des anticorps spécifiques du contaminant à détecter,
b) on évacue lesdits liquides,
c) on transfère la phase solide fluide dans une cuve de révélation,
d) on introduit le substrat et le chromogène dans la cuve de révélation,
e) la réaction de révélation est arrêtée,
f) on mesure la densité optique,
g) on évacue la phase solide fluide,
i) éventuellement une autre phase solide fluide est introduite dans la cuve réactionnelle.

6. Procédé de détection selon la revendication 1, caractérisé en ce que les anticorps ou autres éléments de reconnaissance spécifique sont greffés sur le support notamment par adsorption covalente ou non.

7. Procédé de détection selon la revendication 1, caractérisé en ce que la phase solide fluide est formée de billes ou microbilles choisies notamment parmi les matériaux suivants : le polystyrène, le nylon, le verre, le téflon, le polyéthylène, le polypropylène, I'acétate de cellulose, le polyacrylamide.

8. Procédé selon l'une des revendications précédentes, caractérisé en ce que l'échantillon liquide contient un pesticide, notamment l'atrazine.

9. Phase solide fluide formée de billes ou microbilles associées à des anticorps ou autres éléments de reconnaissance spécifique, pour être utilisées dans le procédé selon l'une des revendications 1 à 8.

10. Phase solide fluide selon la revendication 9, caractérisée en ce que les anticorps sont spécifiques de l'atrazine.

11. Dispositif de détection pour mettre en oeuvre le procédé selon l'une des revendications 1 à 8, caractérisé en ce qu'il comprend une cuve réactionnelle (1) pourvue de moyens d'acheminement
(8) de la phase solide fluide (9),
(10) de l'échantillon liquide à tester (11),
(12) du conjugué lié à une enzyme spécifique d'un substrat chromogène (13),
éventuellement (14) d'eau de lavage (15)
pourvue de moyens d'évacuation,
(3) de la phase solide fluide,
(6) du milieu liquide.
et en ce que le dispositif comprend une cuve de révélation (16) pourvue de moyens d'acheminement :
(17) de la phase solide fluide (9),
(19) d'une solution de substrat (20),
(21) d'une solution de chromogène (22),
(23) d'une solution d'arrêt de réaction (24)
de moyens d'évacuation
(25) de la phase solide fluide
(26) du milieu liquide
(18) des moyens de mesure de la densité optique du milieu liquide.

12. Dispositif selon la revendication 11, caractérisé en ce que la phase solide fluide est transférée de la cuve réactionnelle à la cuve de révélation au moyen d'un fond amovible de la cuve réactionnelle.

13. Dispositif selon l'une des revendications 11 et 12, caractérisé en ce que l'évacuation du milieu liquide est assistée d'une pompe péristaltique.
